# EUROPEAN PATENT SPECIFICATION

(11) **EP 0 760 375 B1**
(45) Date of publication and mention of the grant of the patent: **26.11.2003**
(21) Application number: 95917506.8
(22) Date of filing: 02.05.1995
(51) Int. Cl.: C07H 19/04, C07H 19/044, A61K 31/70

(54) **ANTITUMOR INDOLOPYPROLOCARBAZOLE DERIVATIVE**
ANTITUMOR-INDOLOPYROLOCARBAZOLDERIVAT
DERIVE D'INDOLOPYROLOCARBAZOLE ANTITUMORAL

(30) Priority: 09.05.1994 JP 11948394; 03.06.1994 JP 14564894
(43) Date of publication of application: 05.03.1997
(60) Divisional application: 02018235.8
(73) Proprietor: BANYU PHARMACEUTICAL CO., LTD., Chuo-ku, Tokyo 103-8416 (JP)
(72) Inventor: KOJIRI, Katsuhisa Banyu Pharmaceutical Co., Ltd., Ibaraki 300-33 (JP); KONDO, Hisao Banyu Pharmaceutical Co., Ltd., Ibaraki 300-33 (JP); ARAKAWA, Hiroharu Banyu Pharmaceutical Co., Ltd., Ibaraki 300-33 (JP); OHKUBO, Mitsuru Banyu Pharmaceutical Co., Ltd., Ibaraki 300-33 (JP); SUDA, Hiroyuki Banyu Pharmaceutical Co., Ltd., Ibarraki 300-33 (JP)
(74) Representative: Albrecht, Thomas, Dr.
(86) International application number: JP9500868
(87) International publication number: WO95030682

(56) References cited:
- EP-A- 0 397 060
- EP-A- 0 528 030
- EP-A- 0 602 597
- EP-A- 0 769 555
- WO-A-91/18003
- WO-A-93/11145
- JP-A- 63 198 695
- BERGMAN, JAN ET AL: "Synthesis of indolo[2,3-a]pyrrolo[3,4-c]carbazoles by double Fischer indolizations" J. ORG. CHEM. , vol. 54, no. 4, 1989, pages 824-828, XP002185085
- CHEMICAL ABSTRACTS, vol. 125, no. 3, 1996 Columbus, Ohio, US; abstract no. 34036, KOJIRI, KATSUHISA ET AL: "Preparation of antitumor indolopyrrolocarbazole glycosides" XP002185086 -& WO 96 04293 A (BANYU PHARMACEUTICAL CO., LTD., JAPAN) 15 February 1996 (1996-02-15)

## Description

### Technical Field

This invention relates to novel indolopyrrolocarbazole derivatives which are useful in the field of medicine and, more specifically, inhibit the g rowth of tumor cells and thereby exhibit an anti tumor effect and a process for preparing them, and their use.

### Background Art

In the field of cancer chemotherapy, a large number of compounds have already been put to practical use as antitumor agents. However, their activities against various types of tumors are not necessarily satisfactory, and the problem of tolerance of tumor cells to these anti tumor agents complicates their use for clinical purposes [see the Proceedi ngs of the 47th General Meeting of the Japan Cancer Society, pp. 12-15 (1988)].

Under these circumstances, the development of novel cancerocidal substances are invariably desired in the field of cancer therapy. Among others, there is a need for substances which overcome the problem of tolerance to the existing cancerocidal substances and exhibit effectiveness against such types of cancers as cannot be effectively controlled by the existing cancerocidal substances.

In view of the above-described state of the art, the present inventors screened a wide variety of microbial metabolites, found a novel compound BE-13793C having antitumor activity (12,13-dihydro-1,11-dihydroxy-5H-indolo[2,3-a]pyrrolo[3,4-c]carbazole-5,7(6H)-dione), and disclosed it [see Japanese Laid-Open Patent No. 20277/'91 and the Journal of Antibiotics, Vol. 44, pp. 723-728 (1991)].

Thereafter, they created an i ndolopyrrolocarbazole compound having excellent antitumor activity by a chemical modification of BE-13793C, and disclosed it (see International Publication No. WO91/18003 and European Patent Laid-Open No. E P0545195 A1).

A problem to be solved by the present invention is to create a compound having more excellent antitumor activity than the indolopyrrolocarbazole-derived antitumor substances disclosed in the prior patent application (International Publication No. WO91/18003 and European Patent Laid-Open No. EP0545195 A1).

### Disclosure of the Invention

The present inventors have synthesized a large number of indolopyrrolocarbazole derivatives and have examined their antitumor activities, with a view to creating a compound having more excellent antitumor activity than the indolopyrrolocarbazole-derived antitumor compounds which were previously disclosed. As a result, it has now been found that the compounds represented by the following general formula [I] are novel compounds having very excellent antitumor activity, stability and safety.

Thus, the present invention relates to the compounds of the general formula 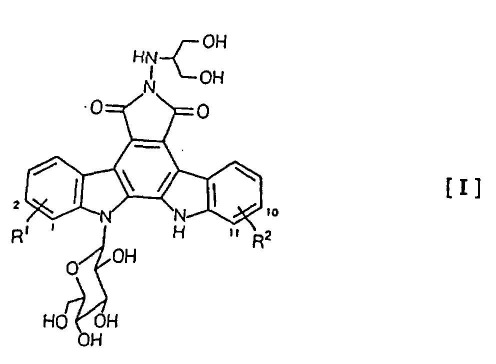 wherein R¹ and R² each represent an OH group, R¹ is located at the 1- or 2-position, R² is located at the 10- or 11-position, R² is located at the 11-position when R¹ is located at the 1-position, and R² is located at the 10-position when R¹ is located at the 2-position, or pharmaceutically acceptable salts thereof, their intermediates, processes for preparing them, and their use.

The compounds of the present invention and their intermediates can be prepared according to the process represented by the following procedure A .

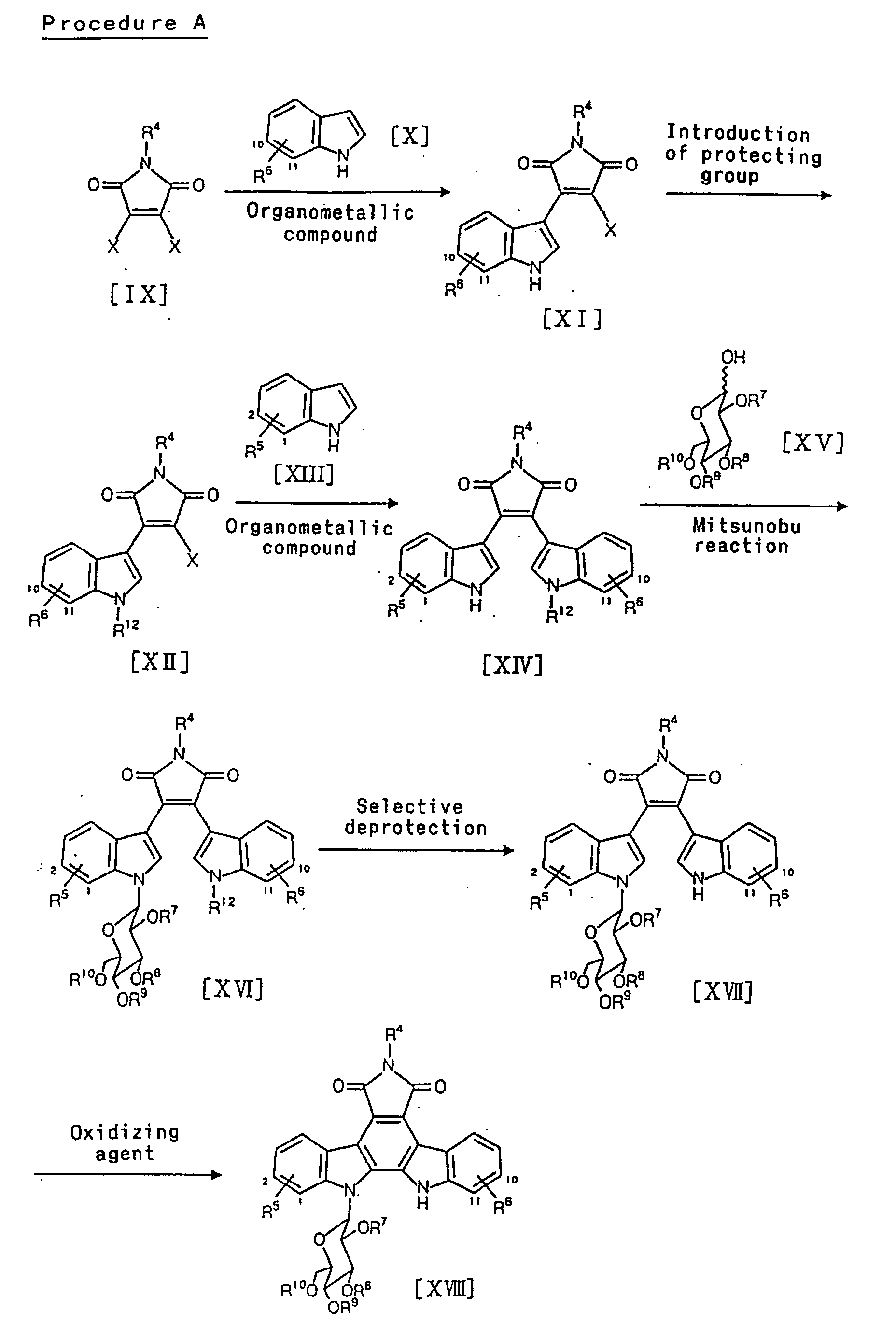

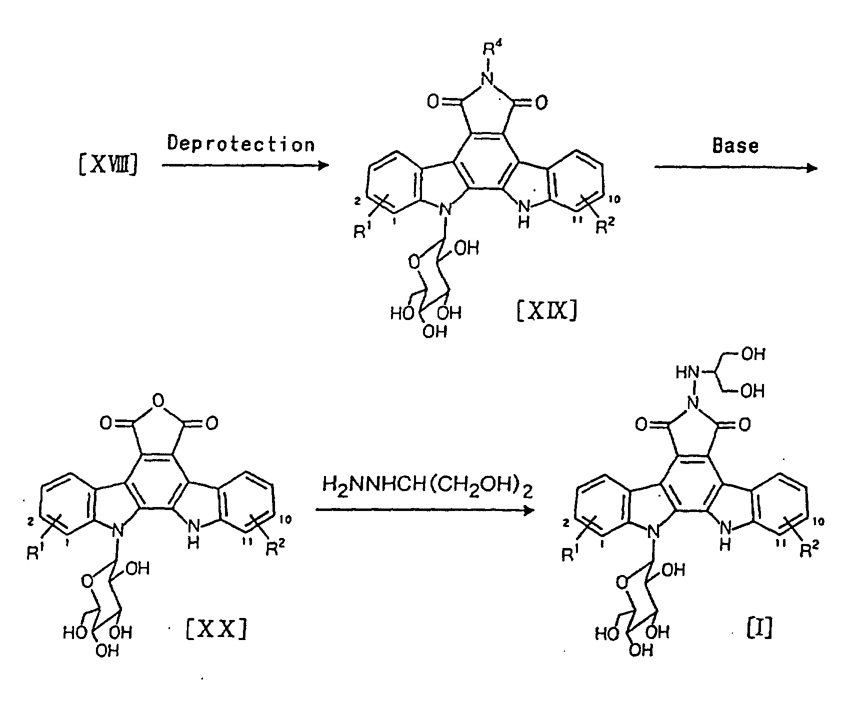

The definitions of the symbols and terms used in procedures A and the claims given late rare as follows.

In the general formulas, R¹ and R² each represent an OH group, provided that R¹ is located at the 1-or 2-position on the ring, R² i s located at the 10- or 11-position on the ring, R² is located at the 11-position when R¹ is located at the 1-position, and R² is located at the 10-position when R¹ is located at the 2-position.

R⁴ represents a hydrogen atom, a lower alkyl group, a benzyloxymethyl group or an aralkyl group. The term "lower alkyl group" means straight-chain or branched alkyl groups of 1 to 6 carbon atoms, such as methyl, ethyl, propyl, sec-propyl, butyl, pentyl and hexyl. The term "aralkyl group" means aralkyl groups of 7 to 12 carbon atoms, such as benzyl, phenethyl and phenylpropyl.

R⁵ and R⁶ each represent a protected OH group, provided that R⁵ is located at the 1- or 2-position on the ring, R⁶ is located at the 10- or 11-position on the ring, R⁶ is located at the 11-position when R⁵ is located at the 1-position, and R⁶ is located at the 10-position when R⁵ is located at the 2-position.

Usable protecting groups include, for example, benzyl, tolyl, p-methoxybenzyl and benzyloxymethyl groups.

R⁷ to R¹⁰ may be the same or different and each represent a protecting group for an OH group . Usable protecting groups include, for example, benzyl, tolyl, p-methoxybenzyl and benzyloxymethyl groups.

R¹² represents a protecting group for the amino group of an indole skeleton. Examples of the protecting group are the same as described above.

X represents a leaving group. Examples thereof include chlorine, bromine and iodine atoms.

The organometallic compound which is used to prepare a compound of the general formula [XI] or the like by reacting a maleimide compound of the general formula [IX] or the like with an indole compound of the general formula [X] or the like can be, for example, an alkyl lithium such as butyl lithium; lithium diisopropylamide; an alkali metal hexaalkyldisilazide such as lithium hexamethyldisilazide, sodium hexamethyldisilazide or potassium hexamethyldisilazide; or a Grignard reagent such as ethylmagnesium bromide or methylmagnesium chloride.

The Mitsunobu reaction is a reaction for forming a glycoside linkage by using an organic phosphine such as triphenylphosphine or tributylphosphine, and an azodicarboxylic acid derivative such as azodicarboxylic acid diethyl ester, azodicarboxylic acid di-tert-butyl ester, azodicarboxylic acid diisopropyl ester, azodicarboxylic acid di-N,N-dimethylamide or azodicarboxylic acid di-N-methylpiperazinamide (see Synthesis, I, 1981, pp. 1-28).

The oxidizing agent which is used to react a compound of the general formula [XVII] or the like having two indole skeletons with an oxidizing agent and thereby convert it to an indolopyrrolocarbazole compound of the general formula [XVIII] or the like can be 2,3-dichloro-5,6-dicyano-1,4-benzoquinone (hereinafter abbreviated as DDQ) , CuCl₂, Cu(OAc)₂, Cu(NO₂)₂, PdCl₂, Pd(OAc)₂, Pd(CF₃COO)₂ or the like.

### Explanation of procedure A

As described above, the reaction of a maleimide compound of the general formula [IX] with an indole compound of the general formula [X] can be carried out with the aid of an alkali metal hexaalkyldisilazide such as lithium hexamethyldisilazide, or a Grignard reagent such as ethylmagnesium bromide. The solvents which can be used in this reaction include toluene, benzene, tetrahydrofuran (THF), dioxane, diethyl ether and the like.

The reaction temperature may usually range from -78°C to 130°C and preferably from -20°C to 110°C.

A compound of the general formula [XII] can be prepared by introducing a protecting group for the amino group of the indole skeleton in a compound of the general formula [XI]. The protective agent used for this purpose can be a halide or acid anhydride corresponding to the aforesaid protecting group. Preferred examples thereof include di-tert-butyl dicarbonate and tert-butyloxycarbonyl chloride.

This reaction is preferably carried out in the presence of a base such as 4-N,N-dimethylaminopyridine. The solvents which can be used in this reaction include toluene, benzene, THF, dioxane, ether and the like. The reaction temperature may usually range from -78°C to 100°C and preferably from -25°C to 25°C.

The preparation of a compound of the general formula [XIV] by reacting the compound of the general formula [XII] with a compound of the general formula [XIII] may be carried out in the same manner as described above for the reaction of the compound of the general formula [IX] with the compound of the general formula [XI].

The reaction of the compound of the general formula [XIV] with a compound of the general formula [XV] can be carried out according to the so-called Mitsunobu reaction. In this reaction, there may be used organic phosphines and azodicarboxylic acid derivatives as described above. Preferred examples of the organic phosphines include tributylphosphine and triphenylphosphine, and preferred examples of the azodicarboxylic acid derivatives include azodicarboxylic acid diethyl ester and azodicarboxylic acid diisopropyl ester.

As the reaction solvent, THF, dioxane, ether and the like may preferably be used. The reaction temperature may usually range from -78°C to 50°C and preferably from -40°C to 20°C.

The deprotection of the amino group of an indole skeleton in a compound of the general formula [XVI] is preferably carried out under conditions which permit selective deprotection. For example, it is preferable to employ acidic or basic conditions which permit the tert-butoxycarbonyl , 2-trimethylsilylethoxymethyl or other group on the amino group to be selectively removed while retaining the other protecting groups.

For example, there may preferably be used acids such as trifluoroacetic acid and HF, and bases such as methylamine, tert-butoxypotassium and tetra-n-butyl ammonium fluoride.

A compound of the general formula [XVIII] can be prepared by oxidatively cyclizing a compound of the general formula [XVII]. The oxidizing agents which can be used in this reaction include, for example, DDQ, CuCl₂, Cu(OAc)₂, Cu(NO₂)₂, PdCl₂, Pd(OAc)₂ and Pd(CF₃COO)₂ as described above. As the reaction solvent, there may be used toluene, methylene chloride, dimethylformamide, dioxane, ether and the like. The reaction temperature may usually range from 0°C to 100°C.

The removal of the protecting groups for the phenolic hydroxyl groups and the glycosyl group in the compound of the general formula [XVIII] can be carried out under acidic conditions or by well-known common hydrogenation reaction or the like.

A compound of the general formula [XX] can be prepared by reacting a compound of the general formula [XIX] with a base. The bases which can be used in this reaction include NaOH, KOH, K₂CO₃, Na₂CO₃, NaHCO₃ and the like. The solvents which can be used therein include water, methanol, ethanol, dimethylformamide and the like. The reaction temperature may usually range from 0°C to the boiling point of the solvent.

A compound of the general formula [I] can be prepared by reacting the compound of the general formula [XX] wi th H₂NNHCH(CH₂OH)₂. The sol vents which can be used in this reaction include methanol, ethanol, THF, dimethylformamide and the like. The reaction temperature may usually range from 0°C to the boiling point of the solvent.

The amount of H₂NNHCH(CH₂OH)₂ used is usually in the range of 1 to 3 molar equivalents based on the compound [XX]. If necessary, this compound may be used in smaller or larger amounts.

After completion of each reaction, the desired product can be isolated and purified according to techniques widely known in the field of organic chemistry (e.g., precipitation, solvent extraction, recrystallization and ch romatography). Moreover, H₂ NNHCH(CH₂OH)₂ can be prepared, for example, according to the procedure described in Example 3.

### Pharmacological tests

The compounds of the general formula [I], which are provided by the present invention, exhibit excellent antitumor effect as demonstrated by the following pharmacological tests.

### (1) Growth-inhibiting activity (CTX) against various types of cancer cells

### Measuring method:

50 µl of a cell culture medium (RPMI-1640 medium containing 10% bovine fetal serum) containing 1 × 10³ mouse leukemia cells (P388), human gastric cancer cells (MKN-45), human pulmonary cancer cells (PC-13) or human rectal cancer cells (DLD-1) was pipetted into the wells of a 96-well microplate, and in cubated at 37°C under 5% CO₂ for 24 hours . Then, 50 µl of a test solution containing each test compound was added, and the culture medium was further incubated at 37°C under 5% CO₂ for 72 hours. After 10 µl of 0.5% Thiazoyl Blue was added to the culture medium, an enzyme reaction was carried out by incubating the culture medium at 37°C under 5% CO₂ for 2 hours. After the reaction was stopped by the addition of 20% sodium dodecyl sulfate (SDS), the culture medium was further incubated at 37°C for 16 hours to dissolve the pigment so formed. Then, the absorbances at 560 nm were measured and compared with that obtained in a control group. The compound of the formula 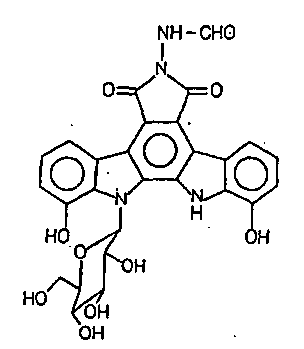 was used as the control compound. The results thus obtained are shown in Table 1.

**Table 1**

| Growth-inhibiting activity against various types of cancer cells | | | | |
|---|---|---|---|---|
| Test compound | CTX(µM) | | | |
| | P388 | MKN-45 | PC-13 | DLD-1 |
| Compound [I-A] | 0.037 | 0.29 | 0.34 | 0.67 |
| Compound [I-B] | 0.0020 | 0.011 | 0.035 | 0.10 |
| Control compound | 0.12 | 0.50 | 1.4 | 73 |

### ( 2) Effect on human gastric cancer MKN-45

A MKN-45 solid tumor which had previously been grown by transplantation under the skin of a nude mouse was minced, and 3 mm cubes of the tumor were transplanted under the skin of mice used for this test. Starting from the time when the transplanted tumor grew to 0.3 cm³, a treatment was carried out by injecting a dose of each test drug into the caudal vein of the mice, once a day, for 5 consecutive days and, after two days' pause, injecting the test drug for 5 days (treatment schedule: 5/w × 2 ) or four times at intervals of 3 or 4 days (treatment schedule: 2/w × 2) . Twenty or thirty-two days after the start of the treatment, the larger diameter (L) and smaller diameter (W) of the tumor were measured, and its volume (V) was determined (V = 1/2 × L W²). The degree of tumor growth inhibition was calculated from this volume, and the total dose at which the tumor growth was inhibited by 75% (GID₇₅ , mg/kg) was determined. The results thus obtained are shown in Table 2 .

**Table 2**

| Effect of the compounds of the present invention on human gastric cancer MKN-45 | | |
|---|---|---|
| Test compound | Treatment schedule | GID₇₅ (mg/kg total) |
| Compound [I-A] | 5/w × 2 | 27 |
| Compound [I-B] | 2/w × 2 | 3.0 |
| Control compound | 5/w × 2 | 170 |

As shown by the results of the above-described pharmacological tests, the compounds provided by the present invention exhibit a more excellent antitumor effect than the control compound.

As is evident from the results of the above-described pharmacological tests, the compounds of the present invention exhibit an excellent antitumor effect and are hence useful as antitumor agents for the prophylaxis or treatment of diseases and, in particular, for the treatment of cancer. When the compounds of the present invention are used for these purposes, they may usually be combined with pharmaceutically acceptable carriers or excipients to make pharmaceutical preparations containing them in effective amounts.

The compounds of the present invention can be used as antitumor agents in various dosage forms. They include, for example, oral preparations such as tablets, capsules, powders, granules and waters; parenteral liquid preparations such as sterilized solutions and suspensions; suppositories; and ointments.

Solid preparations may be made by forming the compounds of the present invention directly into tablets, capsules, granules or powder. However, suitable additives may also be used in combination therewith. Such additives include sugars such as lactose and glucose; starches such as corn, wheat and rice; fatty acids such as stearic acid; inorganic salts such as magnesium aluminate metasilicate and anhydrous calcium phosphate; synthetic polymers such as polyvinyl pyrrolidone and polyalkylene glycol; fatty acid salts such as calcium stearate and magnesium stearate; alcohols such as stearyl alcohol and benzyl alcohol; synthetic cellulose derivatives such as methylcellulose, carboxymethylcellulose, ethylcellulose and hydroxypropylmethylcellulose; and other commonly used additives such as gelatin, talc, vegetable oils and gum arabic.

These solid preparations such as tablets, capsules, granules and powders may generally contain the active ingredient in an amount of 0.1 to 100% by weight and preferably 5 to 100% by weight.

In the case of liquid preparations, the compounds of the present invention may be formed into suspensions, syrups, injections or infusions with the aid of suitable additives commonly used in liquid preparations, such as water, alcohols and vegetable oils (e.g., soybean oil, peanut oil and sesame oil).

Especially when they are parenterally administered by intramuscular, intravenous or subcutaneous injection, suitable solvents include, for example; distilled water for injection, an aqueous solution of lidocaine hydrochloride (for intramuscular injection), physiological saline, an aqueous glucose solution, ethanol, polyethylene glycol, liquids for intravenous injection (e.g., aqueous solutions of citric acid and sodium citrate) and electrolyte solutions (for intravenous drip infusion and intravenous injection), as well as mixtures thereof .

These injections may be prepared not only in previously dissolved form, but also in the form of a powder or mixture with suitable additives for dissolution prior to use. These injections may usually contain the active ingredient in an amount of 0.1 to 10% by weight and preferably 1 to 5% by weight.

Liquid preparations for oral administration, such as suspensions and syrups, may usually contain the active ingredient in an amount of 0.5 to 10% by weight.

The preferred dosages of the compounds of the present invention may vary according to the type of the compound used, the type of the composition prepared, the frequency of use, the site to be treated, the severity of symptoms, the age of the patient, the diagnosis made by the doctor, the type of the tumor, and the like. By way of example, their daily dose for adults may be in the range of 1 to 800 mg for oral administration, and in the range of 0.1 to 500 mg for parenteral administration and preferably for intravenous injection. These daily doses may be given at a time or in 2 to 5 divided doses, depending on the method of administration and the severity of symptoms. Alternatively, they may be administered intermittently, for example, every second or third day.

The present invention is more specifically explained with reference to the following examples.

### Example 1

### Preparation of the compound represented by the formula

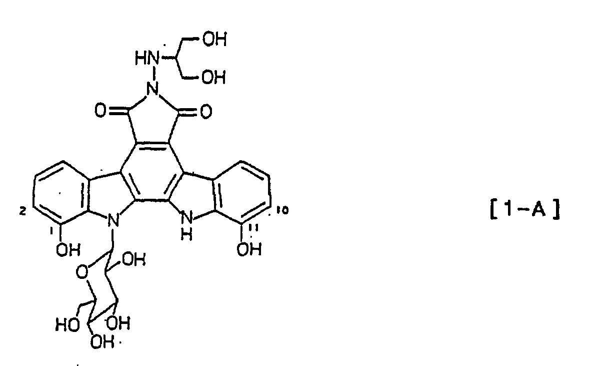

This compound was prepared according to a method comprising the following steps 1) to 9).
1) Preparation of the compound represented by the formula 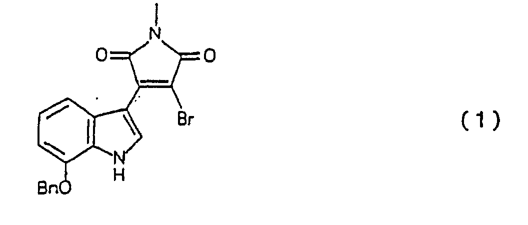 wherein Bn represents a benzyl group and the same will apply hereinafter.
   15 g of 7-benzyloxyindole was dissolved in 150 ml of THF, and 161.3 ml of lithium hexamethyldisilazide (as a 1 M solution in THF) was added thereto. After this mixture was stirred under an atmosphere of nitrogen at 0°C for 30 minutes, 180 ml of a THF solution containing 18.1 g of 2,3-dibromo-N-methylmaleimide was added dropwise thereto over a period of 10 minutes.
   After completion of the addition, the resulting mixture was sti rred at 0°C for 0.5 hour . The reaction mixture was poured into 1 liter of 2N hydrochloric acid and extracted with 2 liters of ethyl acetate. The organic layer was washed with a saturated aqueous solution of sodium hydrogen carbonate and then a saturated aqueous solution of sodium chloride, dried and concentrated. The resulting residue was recrystallized from ethyl acetate-hexane to obtain 26.9 g of the desired compound (1) (in a 97% yield).
   HRMS (m/z): found 410.0273, calcd 410.0248 [as C₂₀H₁₅N₂O₃Br]
   IR (KBr, cm⁻¹): 1705, 1628, 1576, 1433, 1383, 1259, 1247, 1076, 762, 739.
   ¹H-NMR (300 MHz, CDCl₃, δ ppm): 9.03(1H, brs), 7.94(1H, d, J=3.0Hz), 7.64(1H, d, J=8.0Hz), 7.30-7.53(5H, m), 7.15(1H, t, J=8.0Hz), 6,82(1H, d, J=8.0Hz), 5.22(2H, s), 3.16(3H, s).
2) Preparation of the compound represented by the formula 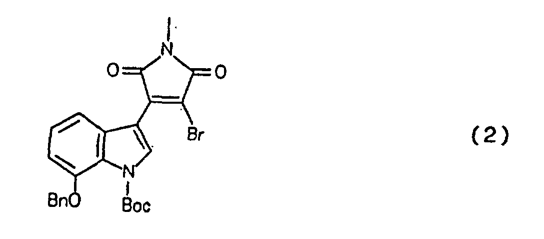 wherein Boc represents a tert-butoxycarbonyl group and the same will apply hereinafter.
   29 g of the compound (1) obtained in Example 1-1), 1 69 g of di-tert-butyl dicarbonate and 136 mg of 4-N,N-dimethylaminopyridi ne were dissolved in 200 ml of THF, and this solution was stirred at room temperature for 1 hour. After the reaction mixture was concentrated, the resul ting residue was purified by silica gel chromatography (chloroform) and then recrystallized from chloroform-ethyl acetate-hexane to obtain 32.9 g of the desired compound (2) (in a 92% yield) .
   IR (KBr, cm⁻¹): 1765, 1712, 1438, 1369, 1261, 1228, 1149, 739.
   HRMS (m/z): found 510.0815, calcd 510.0790 [as C₂₅H₂₃N₂O₅Br]
   ¹H-NMR (300 MHz, CDCl₃, δ ppm): 8.04(1H, s), 7.20-7.62(7H, m), 6.95(1H, d, J=7.9Hz), 5.23(2H, s), 3.18(3H, s) , 1 .53(9H, s) .
3) Preparation of the compound represented by the formula 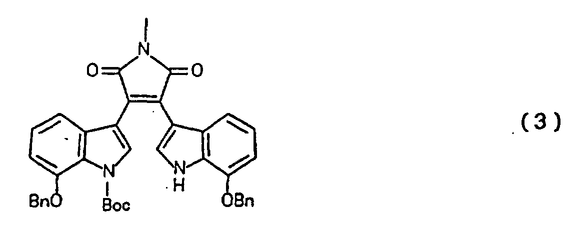
   107.2 mg of 7-benzyloxyindole was dissolved in 3 ml of THF, and 0.48 ml of lithium hexamethyldisilazide (as a 1 M solution in THF) was added thereto. After this mixture was stirred under an atmosphere of nitrogen at 0°C for 15 minutes, 2 ml of a THF solution containing 102.2 mg of the compound (2) obtained in Example 1-2) was added dropwise thereto over a period of 20 minutes. After completion of the addition, the resulting mixture was sti rred at room temperature for 0.5 hour. The reaction mixture was poured into 10 mL of 2N hydrochloric acid and extracted with 30 mL of ethyl acetate. The organic layer was washed with water, a saturated aqueous solution of sodium hydrogen carbonate and then a saturated aqueous solution of sodium chloride, dried and concentrated. The resulting residue was purified by silica gel chromatography (hexane-ethyl acetate = 4 : 1) to obtain 112.7 mg of the desired compound (3) (in a 86% yield) .
   HRMS (m/z): found 653.2529, calcd 653.2526 [as C₄₀H₃₅N₃O₆]
   IR (KBr, cm⁻¹): 1759, 1734, 1579, 1498, 1430, 1261, 1217, 1149, 752, 733.
   ¹H-NMR (300 MHz, CDCl₃, δ ppm): 8.78(1H, brs), 7.90(1H, s), 7.75(1H, s), 7.29-7.52(10H, m), 6.58-6.82(6H, m), 5.17(2H, s), 5.15(2H, S), 3.19(3H, s), 1.53(9H, s).
4) Preparation of the compound represented by the formula 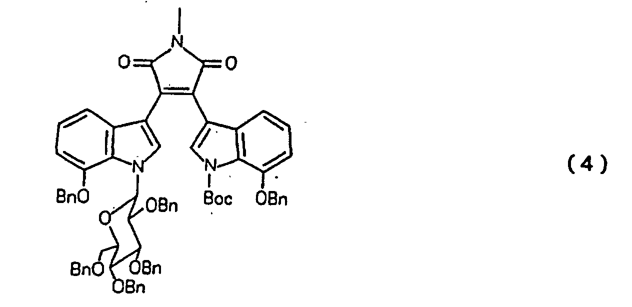
   300 mg of the compound (3) obtained in Example 1-3), 746. 2 mg of 2,3,4,6-O-tetrabenzyl-D-glucopyranose and 543 mg of triphenylphosphine were dissolved in 15 ml of THF, and 0.419 ml of azodicarboxylic acid diisopropyl ester was added thereto at -78°C. This mixture was stirred for 3 hours, during which time its temperature was gradually raised to room temperature. The reaction mixture was partitioned between 40 ml of ethyl acetate and 20 ml of 2N hydrochloric acid. The organic layer was washed with water, a saturated aqueous solution of sodium hydrogen carbonate, water and then a saturated aqueous solution of sodium chloride, dried and concentrated. The resulting residue was purified by silica gel chromatography (toluene-ethyl acetate = 50 : 1) to obtain 459.3 mg of the desired compound (4) (in a 85% yield).
   HRMS (m/z): found 1175.4950, calcd 1175.4932 [as C₇₄H₆₉N₃O₁₁]
   IR (KBr, cm⁻¹): 1759, 1701, 1579, 1454, 1440, 1384, 1358, 1259, 1232, 1149, 1087, 752, 735, 662.
   ¹H-NMR (300 MHz, CDCl₃, δ ppm): 8.19(1H, s), 7.91(1H, s), 7.45(2 H, d, J=6.6Hz), 6.96-7.39(25H, m), 6.54-6.71(7H, m), 6.48(2H, dd, J=1.6, 8.5Hz), 6.43(1H, d, J=8.9Hz), 5.13(2H, s), 5.02(1H, d, J=11.4Hz), 4.90(1H, d, J=10.7Hz), 4.84(1H, d, J=10.8Hz), 4.83(1Hd, J=11.4Hz), 4.80(1H, d, J=10.8Hz), 4.60(1H, d, J=12.7Hz), 4.59(1H, d, J=10.8Hz), 4.53(1H, d, J=11.4Hz), 4.39(1H, d, J=9.9Hz), 3.80(1H, t, J=8.9Hz), 3.64-3.76(4H, m), 3.56(1H, t, J=9.1Hz), 3.38-3.46(1H, m), 3.19(3H, s), 1.53(9H, s).
5) Preparation of the compound represented by the formula 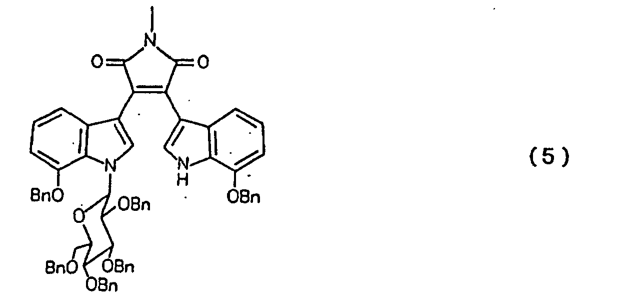
   459.3 mg of the compound (4) obtained in Example 1-4) was dissolved in 20 ml of methylamine (as a 40% solution in methanol), and this solution was stirred at room temperature for 30 minutes. After the reaction mixture was concentrated, the resulting residue was purified by silica gel chromatography (hexane-ethyl acetate = 4 : 1) to obtain 395. 2 mg of the desired compound (5) (in a 94% yield).
   HRMS (m/z): found 1075.4445, calcd 1075.4408 [as C₆₉H₆₁N₃O₉]
   IR (KBr, cm⁻¹): 1697, 1577, 1569, 1497, 1454, 1436, 1257, 1083, 752, 753, 696.
   ¹H-NMR (300 MHz, CDCl₃, **δ** ppm): 8.61(1H, brs), 8.07(1H, s), 7.56(1H, d, J=2.7Hz) , 6.95-7.50(27H, m), 6.85(1H, d, J=7.2Hz), 6.40-6.70(9H, m), 5.13(2H, s), 5.06(1H, d, J=11.1Hz), 4.91(1H, d, J=11.1Hz), 4.90(1H, d, J=11.1Hz), 4.84(1H, d, J=9.6Hz), 4.80(1H, d, J=11.1Hz), 4.48-4.62(3H, m), 4.41(1H, d, J=10.3Hz), 3.64-3.83(4H, m), 3.57(2H, t, J=8.97Hz), 3.40-3.48(1H, m), 3.18(3H, m).
6) Preparation of the compound represented by the formula 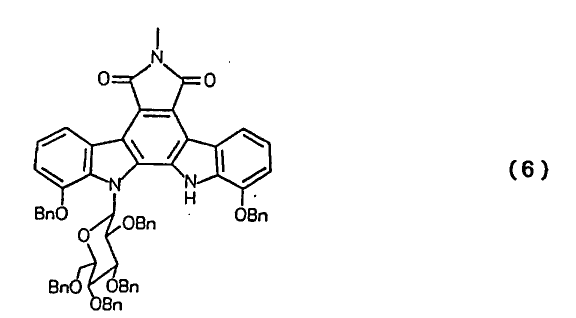
   52 mg of the compound (5) obtained in Example 1-5) was dissolved in 2.5 ml of DMF, and 49.9 mg of palladium tri flouroacetate was added thereto. This mixture was stirred at 90°C for 3 hours. The reaction mixture was partitioned between ethyl acetate and 2N hydrochloric acid. The organic layer was washed with water, a saturated aqueous solution of sodium hydrogen carbonate, water and then a saturated aqueous solution of sodium chloride, dried and concent rated. The resulting residue was purified by silica gel chromatography (hexane-ethyl acetate = 4 : 1) to obtain 26 mg of the desired compound (6) (in a 50% yield).
   HRMS (m/z): found 1073.4269, calcd 1073.4251 [as C₆₉H₅₉N₃O₉]
   IR (KBr, cm⁻¹): 2617, 1699, 1581, 1377, 1257, 1097, 1072, 754, 696.
   ¹H-NMR (300 MHz , CDCl₃, δ ppm ) : 10.55(1H, s), 9.08(1H, d, J=7.3Hz), 8.87(1H, d, J=8.3Hz), 6.90-7.51(31H, m), 6.86(2H, t, J=7.6Hz), 6.17(2H, d, J=6.9Hz), 5.30(1H, d, J=11.5Hz), 5.20(2H, d, J=11.5Hz), 5.14(1H, d, J=11.5Hz), 4.73(1H, d, J=10.9Hz), 4.64(1H, d, J=10.9Hz), 4.59(1H, d, J=11.0Hz), 4.57(1H, d, J=13.1Hz), 4.52(1H, d, J=13.1Hz), 4.10(1H, d, J=11.0Hz), 4.00(1H, t, J=9.1Hz), 3.83(1H, d, J=9.6Hz), 3.52-3.76(5H, m), 3.49(3H, s), 2.95(1H, d, J=9.6Hz).
7) Preparation of the compound represented by the formula 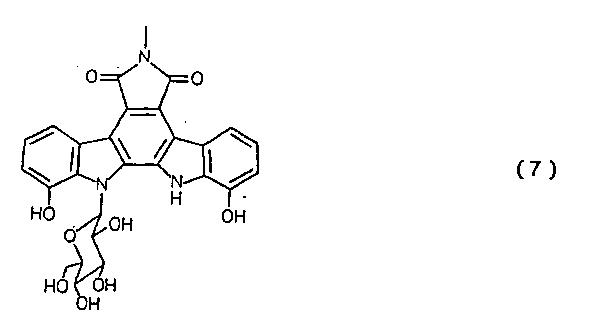
   270 mg of the compound (6) obtained in Example 1-6) was dissolved in 15 ml of chloroform-methanol (1 : 1), and a catalytic amount of palladium black was added thereto. This mixture was stirred under an atmosphere of hydrogen for 4 hours. After the catalyst was filtered off, the filtrate was concentrated. The resulting residue was recrystallized from methanol-chloroform-hexane to obtain 130 mg of the desired compound (7) (in a 98% yield).
   HRMS (m/z): found 533.1419, calcd 533.1434 [as C₂₇H₂₃N₃O₉]
   IR (KBr, cm⁻¹): 3371, 1741, 1638, 1587, 1577, 1387, 1321, 1261, 1238, 1081, 754.
   ¹H-NMR (300 MHz , DMSO-d₆, δ ppm): 10.89(1H, s), 10.34(1H, s), 9.95(1H, s), 8.71(1H, d, J=7.7Hz), 8.53(1H, d, J=7.7Hz), 7.18(2H, t, J=7.7Hz), 7.05(1H, d, J=9.1Hz), 7.01(1H, d, J=7.7Hz), 6.99(1H, d, J=7.7Hz), 4.50-5.80(4H, br), 3.95-4.08(2H, m), 3.58-3.80(3H, m), 3.39(1H, dd, J=8.6, 9.1Hz), 3.18(3H, s).
8) Preparation of the compound represented by the formula 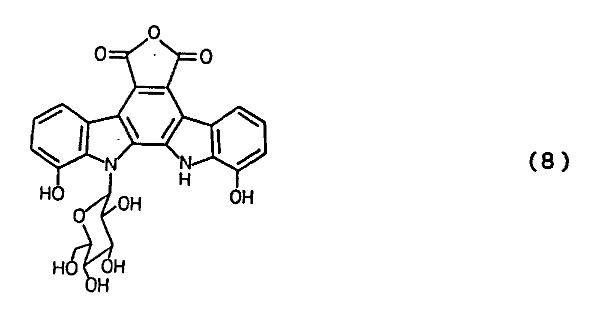
   70 mg of the compound (7) obtained in Example 1-7) was dissolved in 2 ml of a 10% aqueous solution of potassium hydroxide, and this solution was stirred at room temperature for 0.5 hour. The reaction mixture was neutralized by the addition of 1 ml of 2N hydrochloric acid, and then extracted with methyl ethyl ketone-ethyl acetate (1:1). The organic layer was washed with a saturated aqueous solution of sodium chloride, dried and concentrated. The resulting residue was washed with dichloromethane to obtain 65 mg of the title compound (8) (in a 95% yield).
   HRMS (m/z): found 520. 1117, calcd 520.1118 [as C₂₆H₂₀N₂O₁₀]
   IR (KBr, cm⁻¹): 3353, 1816, 1743, 1587, 1388, 1249, 1072, 800, 748, 609.
   ¹H-NMR (300 MHz, DMSO-d₆ , δ ppm) : 11.11(1H, s), 10.52(1H, s), 10.13(1H, s), 8.51(1H, d, J=7.6Hz), 8.36(1H, d, J=7.6Hz), 7.40(1H, d, J=7.8Hz), 7.20(1H, d, J=7.8Hz), 7.09(1H, d, J=8.0Hz), 7.06( 2H, dd, J=7.6, 7.8Hz), 5.32(1H, dd, J=4.9, 5.1Hz), 5.24(1H, d, J=5.4Hz), 4.95(1H, d, J=4.6Hz), 3.95-4.10(2H, m), 3.76(1H, m), 3.56-3.70(2H, m), 3.42(1H, m).
9) 100 mg of the compound (8) obtained in Example 1-8) was dissolved in 10 ml of DMF, and 61 mg of 2-hydrazino-1,3-propanediol was added thereto. This mixture was stirred at 80°C for 1 hour. After the reaction mixture was concentrated, the resulting residue was developed with Sephadex LH-20 and eluted with methanol to obtain 89 mg of the title compound [I-A] (in a 77% yield) .
   HRMS (m/z): found 609. 1826, calcd 609.1833 [as C₂₉H₂₈N₄O₁₁]
   IR (KBr, cm⁻¹): 3309, 1695, 1567, 1540, 1521, 1456, 1417, 1398, 1087, 609.
   ¹H-NMR (300 MHz, DMSO-d₆, δ ppm) : 10.91(1H, brs), 10.30(1H, brs), 9.90(1H, s), 8.70(1H, d, J=8.0Hz), 8.52(1H, d, J=7.9Hz), 7.16-7.21(2H, m), 6.98-7.05(3H, m), 5.59(1H, d, J=2.3Hz), 5.41(1H, d, J=5.7 Hz), 5.20-5.40(2H, m), 5.20(1H, d, J=5.3Hz), 4.90(1H, br), 4.50-4.60(3H, m), 3.98-4.15(2H, m), 3.35-3.80(7H, m).

### Example 2

### Preparation of the compound [I-B] represented by the formul a

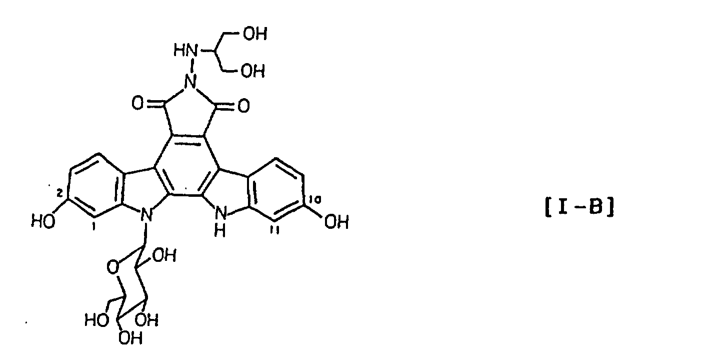

This compound was prepared according to a method comprising the following steps 1) to 9).
1) Preparation of the compound represented by the formula 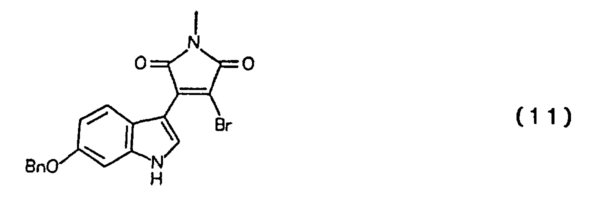
   284 g of 6-benzyloxyindole was dissolved in 3 liters of THF, and 2.7 liters of lithium hexamethyldisilazide (as a 1M solution in THF) was added thereto. After this mixture was stirred under an atmosphere of nitrogen at -10°C for 45 minutes, 3 liters of a THF solution containing 340 g of 2,3-dibromo-N-methylmaleimide was added dropwise thereto over a period of 1 hour.
   After completion of the addition, the resulting mixture was stirred at 0°C for 15 minutes. The reaction mixture was poured into 10 liters of 2N hydrochloric acid and extracted with 30 liters of ethyl acetate. The organic layer was washed with a saturated aqueous solution of sodium hydrogen carbonate and then a saturated aqueous solution of sodium chloride, dried and concentrated. The resulting re sidue was recrystallized from methanol to obtain 482 g of the desi red compound (11) (in a 93% yield).
   HRMS (m/z): found 410.0292, calcd 410.0266 [as C₂₀H₁₅N₂O₃Br]
   IR (KBr, cm⁻¹): 3330, 3318, 1762 , 1701 , 1606 , 1511, 1450, 1165, 1135, 1041, 794.
   ¹H-NMR (300 MHz, CDCl₃, δ ppm): 8.60(1H, brs), 7.96(1H, d, J=8.1Hz), 7.94(1H, d, J=2.5Hz), 7.33-7.47(5H, m), 7.00(1H, dd, J=2.5, 8.8Hz), 6.97(1H, d , J=2.5Hz), 5.13(2H, s) , 3.16(3H, s) .
2) Preparation of the compou nd represented by the formula 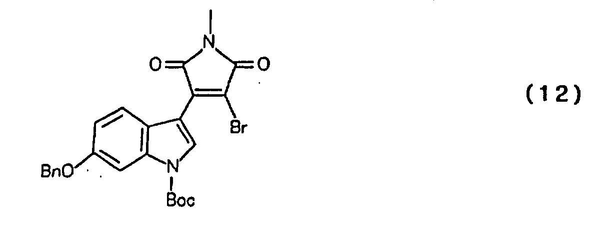
   1.00g of the compound (11) obtained in Example 2-1), 637 mg of di-tert-butyl dicarbonate and 3 mg of 4-N,N-dimethylaminopyridine were dissolved in 200 ml of THF, and this solution was stirred at room temperature for 1 hour. After the reaction mixture was concentrated, the resulting residue was recrystallized from ethyl acetate-hexane to obtain 1.18 g of the desired compound (12) (in a 96% yield).
   IR (KBr, cm⁻¹): 1740, 1714, 1614, 1527, 1487, 1443, 1373, 1227, 1153.
   HRMS (m/z) : found 510.0771, calcd 510.0791 [as C₂₅H₂₃N₂O₅Br]
   ¹H-NMR (300 MHz, CDCl₃, δ ppm): 8.10(1H, s), 7.91(1H, d, J=2.3Hz), 7.73(1H, d, J=8.9Hz), 7.34-7.50(5H, m), 7.03(1H, dd, J=2.3, 8.5Hz), 5.16(2H, s), 3.18(3H, s), 1.68(9H, s) .
3) Preparation of the compound represented by the formula 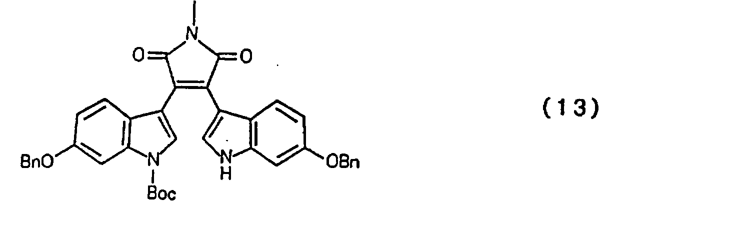
   218.4 mg of 6-benzyloxyindole was dissolved in 20 ml of THF, and 2 .35 ml of lithium hexame thyldisilazide (as a 1M solution in THF) was added thereto. After this mixture was stirred under an atmosphere of nitrogen at 0°C for 15 minutes, 10 ml of a THF solution containing 500 mg of the compound (12) obtained in Example 2-2) was added dropwise there to over a period of 10 minutes. After completion of the addition, the resulting mixture was stirred at room temperature for 0.5 hour. The reaction mixture was poured into 100 mL of 2N hydrochloric acid and extracted with 400 mL of ethyl acetate. The organic layer was washed with water, a saturated aqueous solution of sodium hydrogen carbonate and then a saturated aqueous solution of sodium chloride, dried and concentrated. The resulting residue was recrystallized from toluene-hexane to obtain 580 mg of the desired compound (13) (in a 91% yiel d) .
   HRMS (m/z): found 653.2556, calcd 653.2526 [as C₄₀H₃₅N₃O₆]
   IR (KBr, cm⁻¹): 1740, 1701, 1646, 1623, 1543, 1445, 1 155 .
   ¹H-NMR (300 MHz, CDCl₃, δ ppm) : 8.41(1H, brs), 7.97(1H, s), 7.84(1H, brs), 7.68(1H, brs), 7.16-7.43(10H, m), 6.98(1H, d, J=9.2Hz), 6.85(1H, brs), 6.74(1H, d, J=9.2Hz), 6.58(1H, d, J=9.2Hz), 6.52(1H, d, J=9.2Hz), 5.05(2H, s), 5.02(2H, s), 3.19(3H, s), 1.67(9H, s).
4) Preparation of the compound represented by the formula 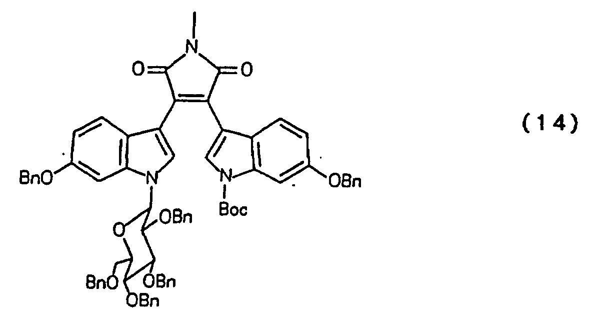
   50 mg of the compound (13) obtained in Example 2-3), 186 mg of 2,3,4,6-O-tetrabenzyl-D-glucopyranose and 90 mg of triphenylphosphine were dissolved in 3 ml of THF, and 0.054 ml of azodicarboxylic acid diethyl ester was added thereto at 0°C. This mixture was stirred for 1 hour. The reaction mixture was partitioned between 40 ml of ethyl acetate and 20 ml of 2N hydrochloric acid. The organic layer was washed with water, a saturated aqueous solution of sodium hydrogen carbonate, water and then a saturated aqueous solution of sodium chloride, dried and concentrated. The resulting residue was purified by silica gel chromatography (hexane-ethyl acetate = 4 : 1) to obtain 36.4 mg of the desired compound (14) (in a 62% yield).
   HRMS (m/z): found 1175.4955, calcd 1175.4932 [as C₇₄H₆₉N₃O₁₁]
   IR (KBr, cm⁻¹): 2360, 1736, 1701, 1616, 1543, 1489, 1454, 1363 , 1219, 1153.
   ¹H-NMR (300 MHz, CDCl₃, δ ppm) : 8.03(1H, s), 7.95(1H, s), 7.78-7.82(1H, m), 7.04-7.38(30H, m), 6.84(1H, d, J=8.7Hz), 6.76-6.84(1H, m), 6.79(1H, d, J=8.9Hz), 6.32(1H, dd, J=2.2, 8.9Hz), 6.28(1H, dd, J=2.4, 8.7Hz) , 5.33(1H, d, J=8.7Hz), 4.82-4.94(7H, m), 4.67(1H, d, J=10.6Hz), 4.63(1H, d, J=12.1 H), 4.55(1H, d, J=12.1Hz), 4.10(1H, d, J=10.2Hz), 3.69-3.95(6H, m), 3.40(1H, d, J=10.2Hz), 3.20(3H, s), 1.66(9H, s).
5) Preparation of the compound represented by the formula 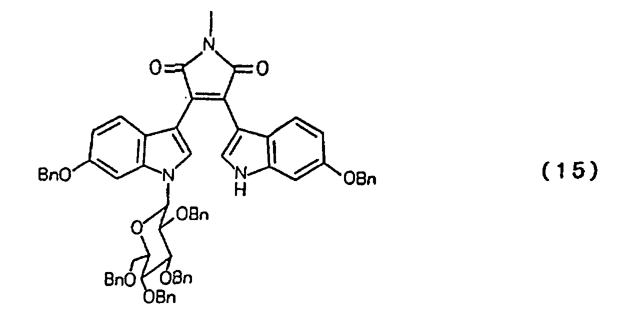
   14.1 mg of the compound (14) obtained in Example 2-4) was dissolved in 5 ml of methylamine (as a 40% solution in methanol), and this solution was stirred at room temperature for 30 minutes. After the reaction mixture was concentrated, the resulting residue was purified by silica gel chromatography (hexane-ethyl acetate = 7 : 3) to obtain 12.3 mg of the desired compound (15) (in a 96% yield).
   HRMS (m/z): found 1075.4392, calcd 1075.4408 [as C₆₉H₆₁N₃O₉]
   I R (KBr, cm⁻¹): 3311, 3030 , 2927 , 1697, 1621, 1533, 1454, 1385, 1159, 1093.
   ¹H-NMR (300 MHz, CDCl₃, δ ppm): 8.29(1H, d,J=2.7Hz), 7.96(1H, s), 7.52(1H, d, J=2.7Hz), 7.19-7.40(25H, m), 7.03-7.19(5H, m) , 6.78-6.84(3H, m), 6.67(1H, d, J=8.8Hz), 6.45(1H, dd, J=2.2, 8.8Hz), 6.34(1H, dd, J=2.2, 8.8Hz), 5.34(1H, d, J=8.7Hz), 4.82-4.94(7H, m), 4.67(1H, d, J=10.7Hz), 4.62(1H, d, J=12.2Hz) , 4.53(1H, d, J=12.2Hz), 4.12(1H, d, J=10.2Hz), 3.67-3.98(7H, m), 3.18(3H, m).
6) Preparation of the compound represented by the formula 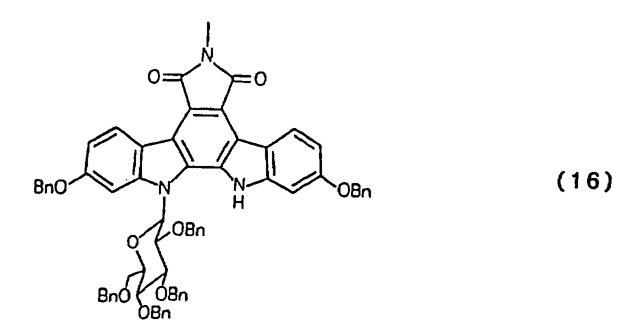
   52 mg of the compound (15) obtained in Example 2-5), 26.8 mg of copper(II) chloride and 50 mg of molecular sieve were dissolved in 1 ml of methyl ethyl ketone, and this solution was sti rred at room temperature for 2 hours. After the reaction mixture was filtered through celite, the filtrate was concentrated. The resulting residue was purified by silica gel chromatography (dichloromethane) to obtain 42 mg of the desired compound (16) (in a 84% yield).
   HRMS (m/z): found 1073.4237, calcd 1073.4251 [as C₆₉H₅₉N₃O₉]
   IR (K Br, cm⁻¹): 3311, 3030, 2927, 1697, 1621, 1533, 1454, 1385, 1159, 1093.
   ¹H-NMR (300 MHz, CDCl₃, **δ** ppm) : 10.6(1H, s), 9.24(1H, d, J=9.5Hz), 9.13(1H, d, J=9.5Hz), 7.07-7.50(29H, m), 6.98-7.03(1H, m), 6.83-6.91(2H, m), 6.18-6.22(2H, m), 5.84(1H, d, J=8.9Hz), 5.12-5.22(2H, m), 5.18(1H, d, J=11.5Hz), 5.08(1H, d, J=11.5Hz), 4.97(1H, d, J=10.7Hz), 4.89(1H, d, J=10.7Hz), 4.84(1H, d, J=10.7Hz), 4.74(1H, d, J=13.0Hz), 4.67(1H, d, J=10.7Hz), 4.56(1H, d, J=13.0Hz), 4.32(1H, dd, J=9.6, 9.6Hz), 3.98-4.07(2H, m), 3.82-3.97(3H, m), 3.79(1H, dd, J=2.7, 10.2Hz), 3.33(3H, s), 3.00(1H, d, J=9.7Hz).
7) Preparation of the compound represented by the formula 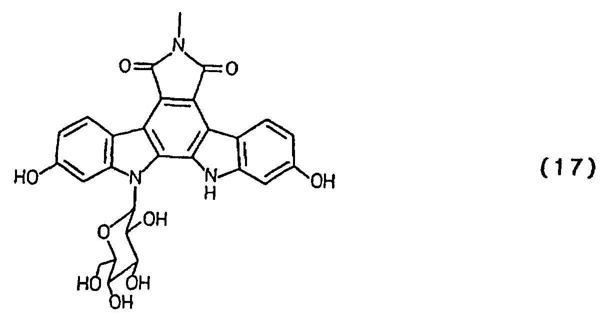
   100 mg of the compound (16) obtained in Example 2-6) was dissolved in 6 ml of chloroform-methanol (2 : 1), and a catalytic amount of palladium black was added thereto. This mixture was stirred under an atmosphere of hydrogen for 2 hours. After the catalyst was filtered off, the filtrate was concentrated. The resulting residue was crystallized from methanol-acetone-ethyl acetate-hexane, developed with Sephadex LH-20, eluted with chloroform-methanol-ethanol-tetrahydrofuran (5 : 2 : 2 : 1), and recrystallized from acetone-methanol-hexane to obtain 43.8 mg of the desired compound (17) (in a 88% yield).
   HRMS (m/z): found 533.1429, calcd 533.1434 [as C₂₁H₂₃N₃O₉]
   IR (KBr, cm⁻¹): 3328, 1733, 1683, 1678, 1540, 1417, 1126, 1081, 611.
   ¹H-NMR (300 MHz, DMSO-d₆, δ ppm): 11.20(1H, s), 9.76(1H, s), 9.74(1H, s), 8.88(1H, d, J=8.6Hz), 8.80(1H, d, J=8.6Hz), 7.18(1H, d, J=2.1Hz), 6.99(1H, d, J=2.1Hz), 6.82(1H, dd, J=2.1, 8.6Hz), 6.80(1H, dd, J=2.1, 8.6Hz), 5.97(1H, d, J=8.9Hz), 5.86(1H, t, J=4.0Hz), 5.33(1H, d, J=4.9Hz), 5.12(1H, d, J=4.3Hz), 4.94(1H, d, J=5.2Hz), 4.02(1H, dd, J=3.0, 10.7Hz) , 3.94(1H, m), 3.78(1H, m), 3.52(2H, m), 3.16(3H, s).
8) Preparation of the compound represented by the formula 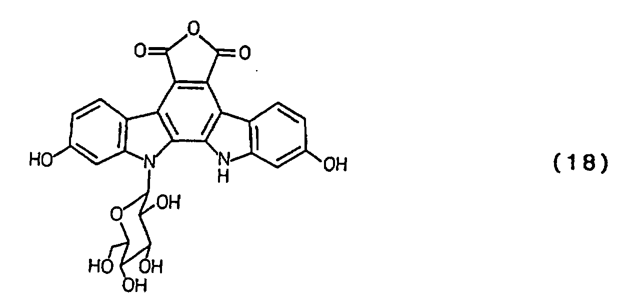
   1.2 g of the compound (17) obtained in Example 2-7) was dissolved in 40 ml of a 10% aqueous solution of potassium hydroxide, and this solution was stirred at room temperature for 1 hour. The reaction mixture was neutralized by the addition of 40 ml of 2N hydrochloric acid, and then extracted with 1 liter of methyl ethyl ketone. The organic layer was washed with a saturated aqueous solution of sodium chloride, dried and concentrated. The resulting residue was recrystallized from acetone-heptane to obtain 1.2 g of the desired compound (18) (in a 100% yield) .
   HRMS (m/z): found 520.1147, calcd 520.1118 [as C₂₆H₂₀N₂O₁₀]
   IR (KBr, cm⁻¹): 3311, 1810, 1739, 1652, 1626, 1558, 1405, 1091, 611.
   ¹H-NMR (300 MHz, DMSO-d₆, **δ** ppm) : 11.4(1H, s), 9.95(1H, s), 9.92(1H, s), 8.69(1H, d, J=7.7Hz), 8.63(1H, d, J=7.7Hz), 7.25(1H, d, J=1.5Hz), 7.03(1H, d, J=1.5Hz), 6.90(1H, dd, J=1.5, 7.7Hz), 6.87(1H, d, J=1.5, 7.7Hz), 6.06(1H, d, J=8.0Hz), 5.95(1H, t, J=4.6Hz), 5.38(1H, d, J=5.1Hz), 5.16(1H, d, J=5.2Hz), 4.99(1H, d, J=5.2Hz), 3.30-4.10(6H, m).
9) 500 mg of the compound (18) obtained in Example 2-8) was dissolved in 50 ml of DMF, and 152 mg of 2-hydrazino-1,3-propanediol was added thereto. This mixture was stirred at 80°C for 1 hour. After the reaction mixture was concentrated, the resulting residue was purified with Sephadex LH-20 (chloroform-methanol-ethanol-water = 5 : 2 : 2 : 1) to obtain 418 mg of the title compound [I-B] (in a 77% yield).
   HRMS (m/z): found 609.1816, calcd 609.1833 [as C₂₉H₂₈N₄O₁₁]
   IR (KBr, cm⁻¹): 3415, 3353, 1749, 1652, 1575,
   1540, 1375, 1197, 6 09 .
   ¹H-NMR (300 MHz, DMSO-d₆ ; δ ppm) : 11.20(1H, s), 9.78(1H, s), 9.75(1H, s), 8.87(1H, d, J=8.6Hz), 8.79(1H, d, J=8.6Hz), 7.18(1H, d, J=2.0Hz), 6.98(1H, d, J=2.0Hz), 6.82(1H, dd, J=2.0, 8.6Hz), 6.80(1H, dd, J=2.0, 8.6Hz), 5.97(1H, d, J=8.3Hz), 5.86(1H, d, J=3.8Hz), 5.55(1H, d, J=2.6Hz), 5.32(1H, d, J=4.6 Hz), 5.11(1H, d, J=5.3Hz), 4.91(1H, d, J=5.1Hz), 4.53(2H, t, J=5.4Hz), 4.02(1H, m), 3.85-3.95(2H, m), 3.78(1H, m), 3.40-3.60(6H, m), 3.20-3.30(1H, m).

### Example 3

### Preparation of the compound represented by the formula

H₂ NNHCH(CH₂ OH)₂ (26)

1) 10.0 g of dihydroxyacetone dimer and 14.7 g of tert-butyl carbazinate were dissolved in 500 ml of ethanol, and this solution was stirred at room temperature for 15 hours. After the reaction mixture was concentrated under reduced pressure, the resulting residue was recrystallized from ethyl acetate to obtain 18.67 g of 2-(tert-butyloxycarbonyl)-hydrazono-1,3-propanediol as a colorless solid.
   ¹H-NMR (300 MHz, DMSO-d₆ , δ ppm) : 1.49(9H, s), 3.92(2H, d, J=5.2Hz), 4.24(2H, d, J=5.0Hz), 4.88(1H, t, J=5.8Hz), 5.61(1H, t, J=5.1Hz), 9.98(1H, brs).
2) 50 ml of a borane-tetrahydrofuran complex was added to 5.00 g of 2-(tert-butyloxycarbonyl)-hydrazono-1,3-propanediol at 0°C , and this mixture was stirred at room temperature for 0.5 hour. 25 ml of 6N hydrochloric acid was added to the reaction mixture, and the resulting mixture was heated under reflux for 1 .5 hours. After the reaction mixture was concentrated under reduced pressure, the resulting residue was adsorbed to Dowex 50Wx4 of the H⁺ type, washed with water, and eluted with 0.5N aqueous ammoni a. After fractions containing the desired product were collected and concentrated under reduced pressure, the resul ting oily material was adsorbed to IRC-50 of the NH₄⁺ type and eluted with water. Fractions containing the desired product were collected and concentrated under reduced pressure to obtain 2.26 g of 2-hydrazino-1,3-propanediol as a colorless solid.
   FAB-MS (m/z): 10 7 (M+H)⁺
   ¹H-NMR (200 MHz, CD₃OD, δ ppm): 2.78(1H, m), 3.50-3.75(4H, m) .

### Exploitability in Industry

The compounds of the present invention have an excellent antitumor effect and are hence useful as antitumor agents in the field of medicine.

## Claims

1. A compound of the general formula 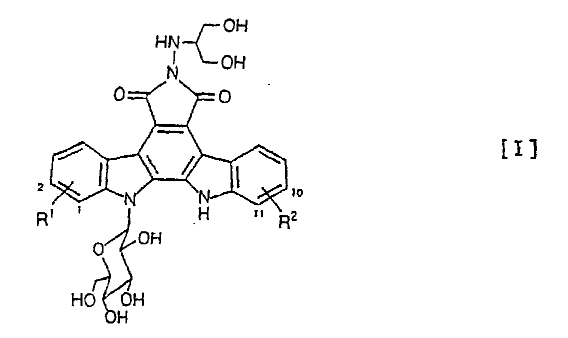 or a pharmaceutically acceptable salt thereof, wherein R¹ and R² each represent an OH group, R¹ is located at the 1- or 2-position, R² is located at the 10- or 11-position, R² is located at the 11-position when R¹ is located at the 1-position, and R² is located at the 10-position when R¹ is located at the 2-position.

2. The compound of the formula 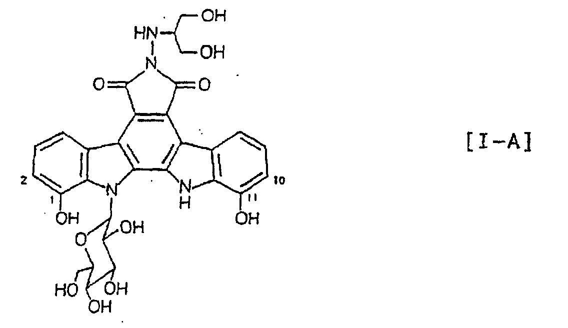 or a pharmaceutically acceptable salt thereof.

3. The compound of the formula 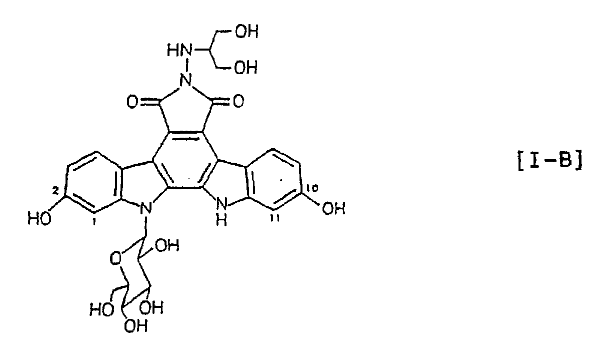 or a pharmaceutically acceptable salt thereof .

4. A process for the preparation of a compound of formula [I] 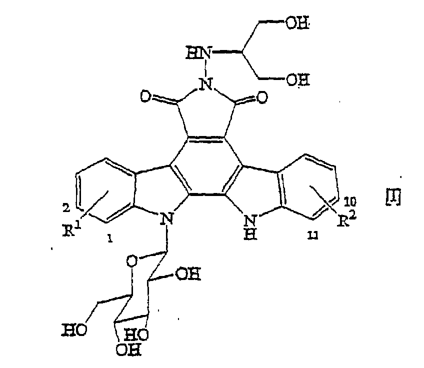 wherein R¹ and R² each represent an OH group, R¹ is located at the 1- or 2-position, R² is located at the 10- or 11-position, R² is located at the 11-position when R¹ is located. at the 1-position, and R² is located at the 10-position when R¹ is located at the 2-position, which comprises:
reacting a compound of formula [IX] 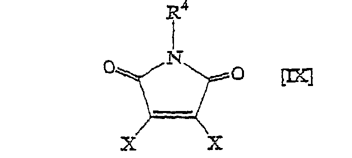 wherein R⁴ represents a hydrogen atom, an alkyl group having 1 to 6 carbon atoms, a benzyloxymethyl group or an aralkyl group, and X represents a leaving group,
with a compound of formula [X] 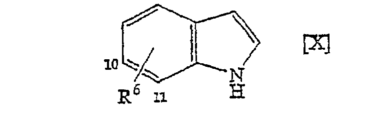 wherein R⁶ represents a protected OH group located at the 10- and 11-position, in the presence of an organometallic compound to form a compound of formula [XI] 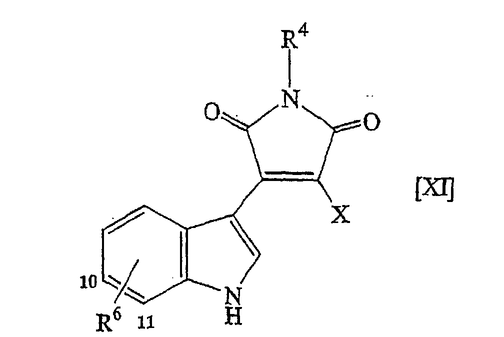 wherein R⁴, R⁶, and X have the same meaning as described above;
protecting the hydrogen atom of the amino group of the indole skeleton to form a compound of formula [XII] 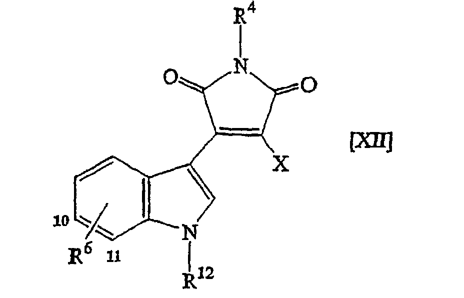 wherein R⁴, R⁶ and X are the same meaning as described above, and R¹² represents a protecting group for the amino group of the indole skeleton;
reacting the compound of formula [XII] with a compound of formula [XIII] 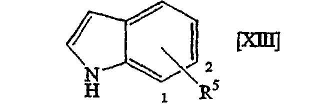 wherein R⁵ represents a protected OH group at the 1- or 2-position, in the presence of an organometallic compound, to form a compound of formula [XIV] 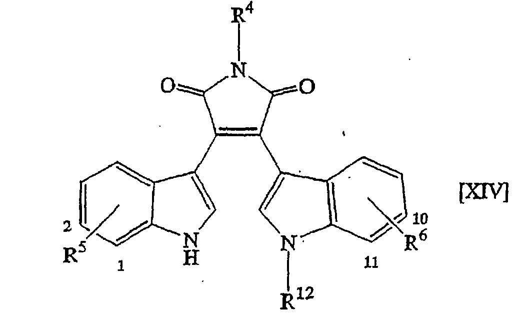 wherein R⁴, R⁵, R⁶ and R¹² are the same meaning as described above, and R⁵ and R⁶ are located at the 1- and 11-positions, respectively, or are located at the 2- and 10-positions, respectively;
reacting the compound of formula [XIV] with a compound of formula [XV] 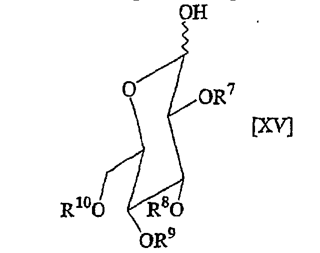 wherein R⁷, R⁸, R⁹ and R¹⁰, may be the same or different and each represents a protecting group for a hydroxyl group, by the Mitsunobu reaction, to form a compound of formula [XVI] 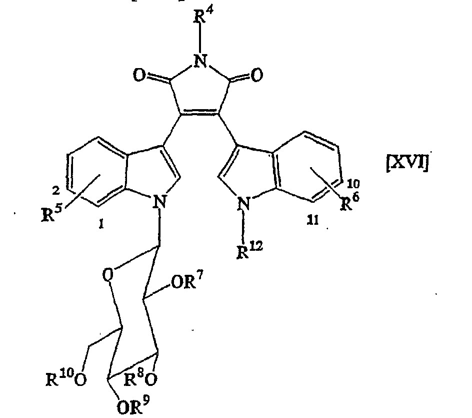 wherein R⁴ to R¹⁰ and R¹² are the same meaning as described above;
removing the protecting group for the amino group of the indole skeleton to form a compound of formula[XVII] 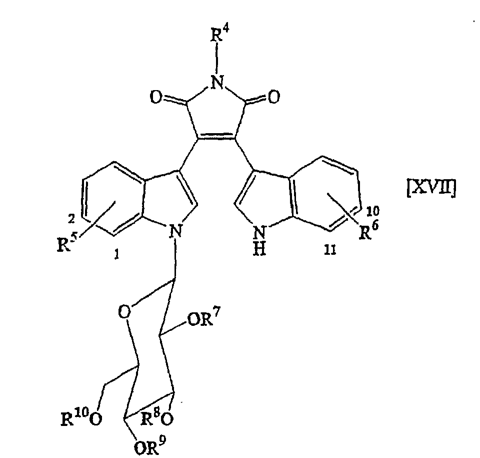 wherein R⁴ to R¹⁰ have the same meaning as described above;
cyclizing the compound of formula [XVII] with the aid of an oxidizing agent to form a compound of formula [XVIII] 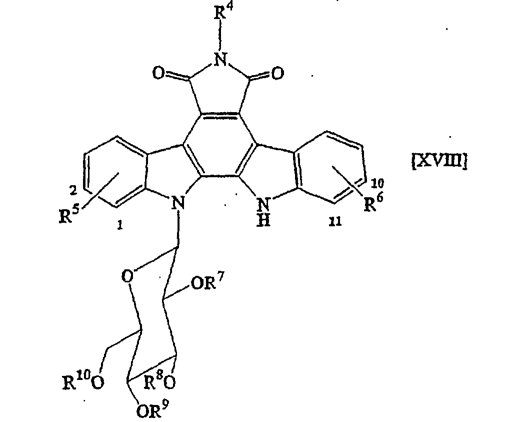 wherein R⁴ to R¹⁰ are the same meaning as described above;
removing the protecting groups for OH groups to form a compound of formula [XIX] 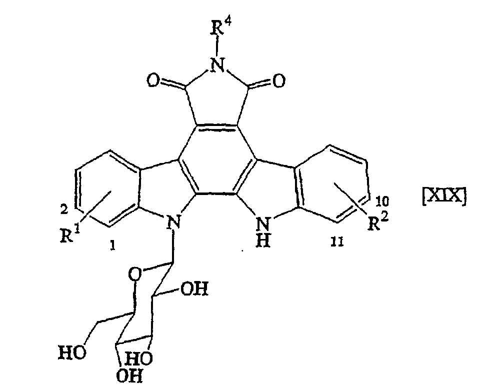 wherein R¹ and R² each represents an OH group; and R¹ and R² are located at the 1-and 11-positions, respectively, or R¹ and R² are located at the 2- and 10-positions, respectively; and R⁴ is the same meaning as described above;
reacting the compound of formula [XIX] with a base to form a compound of formula [XX] 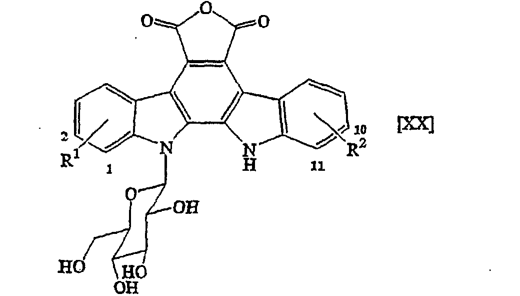 wherein R¹ and R² are the same meaning as described above; and
reacting the compound of formula [XX] with H₂NNHCH(CH₂OH)₂.

5. The compound of the formula
H₂ NNHCH (CH₂OH)₂

6. An antitumor agent containing a compound as claimed in any of claims 1 to 3.

## Patentansprüche

1. Verbindung der allgemeinen Formel 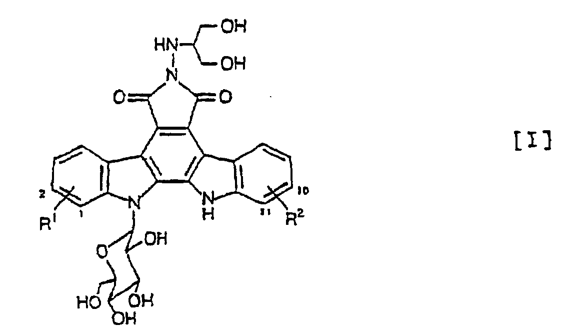 oder ein pharmazeutisch annehmbares Salz davon, worin R¹ und R² je eine OH-Gruppe bedeuten, R¹ an der 1- oder 2-Stellung vorhanden ist, R² an der 10- oder 11-Stellung vorhanden ist, R² an der 11-Stellung vorhanden ist, wenn R¹ an der 1-Stellung vorhanden ist, und R² an der 10-Stellung vorhanden ist, wenn R² an der 2-Stellung vorhanden ist.

2. Verbindung der Formel 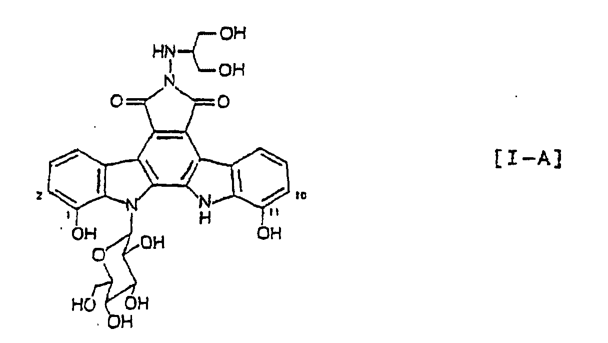 oder ein pharmazeutisch annehmbares Salz davon.

3. Verbindung der Formel 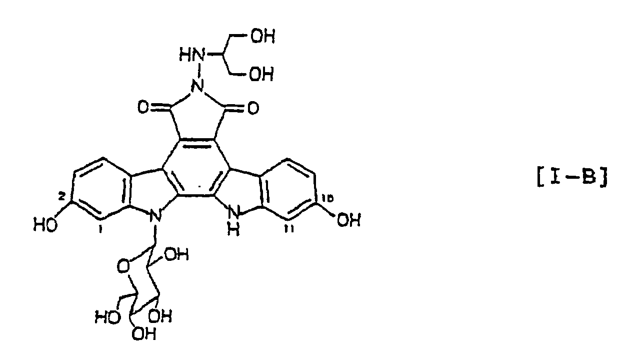 oder ein pharmazeutisch annehmbares Salz davon.

4. Verfahren zur Herstellung einer Verbindung der Formel [I] 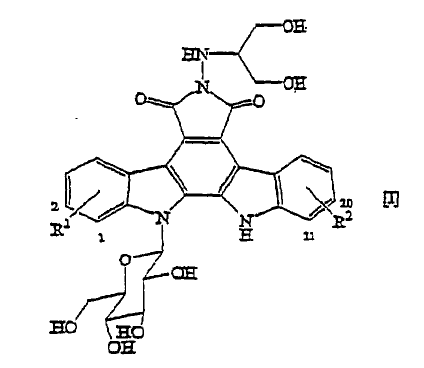 worin R¹ und R² je eine OH-Gruppe bedeuten, R¹ an der 1-oder 2-Stellung vorhanden ist, R² an der 10- oder 11-Stellung vorhanden ist, R² an der 11-Stellung vorhanden ist, wenn R¹ an der 1-Stellung vorhanden ist, und R² an der 10-Stellung vorhanden ist, wenn R¹ an der 2-Stellung vorhanden ist, umfassend:
Umsetzung einer Verbindung der Formel [IX] 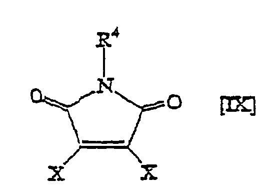 worin R⁴ ein Wasserstoffatom, eine Alkylgruppe mit 1 bis 6 Kohlenstoffatomen, eine Benzyloxymethylgruppe oder eine Aralkylgruppe bedeutet und X eine Austrittsgruppe bedeutet,
mit einer Verbindung der Formel [X] 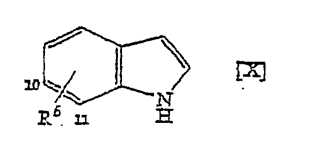 worin R⁶ eine geschützte OH-Gruppe, vorhanden an der 10-und 11-Stellung, bedeutet, in Anwesenheit einer metallorganischen Verbindung unter Bildung einer Verbindung der Formel [XI] 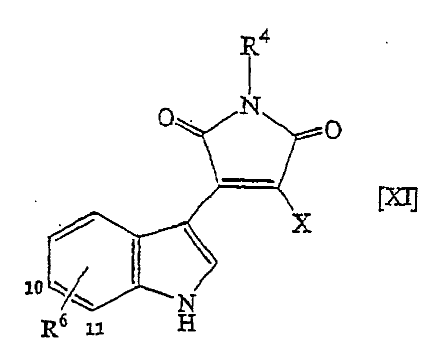 worin R⁴, R⁶ und X die oben gegebenen Definitionen besitzen;
Schützen des Wasserstoffatoms der Aminogruppe des Indolskeletts unter Bildung einer Verbindung der Formel [XII] 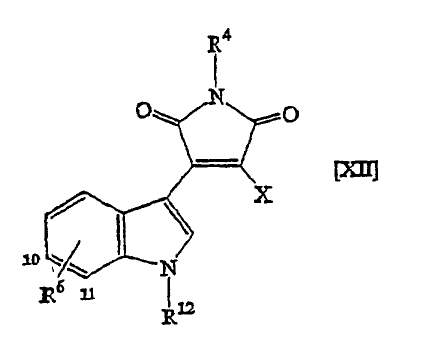 worin R⁴, R⁶ und X die gleichen Bedeutungen wie oben beschrieben besitzen und R¹² eine Schutzgruppe für die Aminogruppe des Indolskeletts bedeutet;
Umsetzung der Verbindung der Formel [XII] mit einer Verbindung der Formel [XIII] 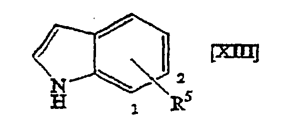 worin R⁵ eine geschützte OH-Gruppe in der 1- oder 2-Stellung bedeutet, in Anwesenheit einer metallorganischen Verbindung, unter Bildung einer Verbindung der Formel [XIV] 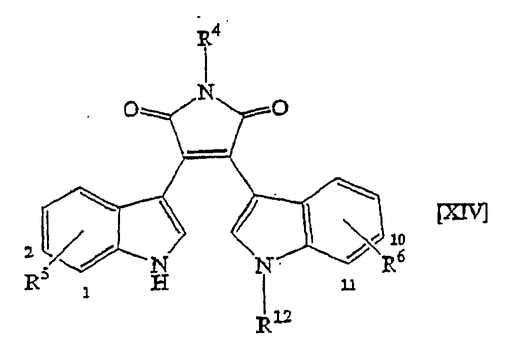 worin R⁴, R⁵, R⁶ und R¹² die gleichen Bedeutungen wie oben beschrieben besitzen, und R⁵ und R⁶ in den 1- bzw. 11-Stellungen vorhanden oder in den 2- bzw. 10-Stellungen vorhanden sind,
Umsetzung der Verbindung der Formel [XIV] mit einer Verbindung der Formel [XV] 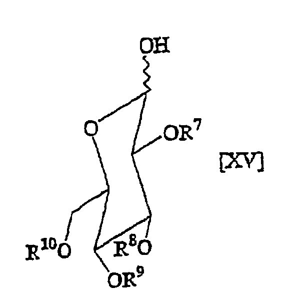 worin R⁷, R⁸, R⁹ und R¹⁰ gleich oder unterschiedlich sein können und je eine Schutzgruppe für eine Hydroxylgruppe bedeuten, durch Mitsunobu-Reaktion unter Bildung einer Verbindung der Formel [XVI] 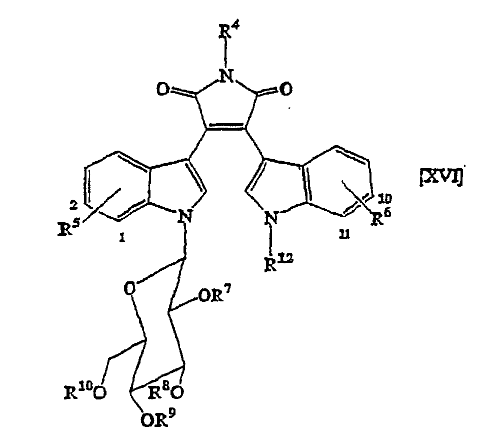 worin R⁴ bis R¹⁰ und R¹² die gleichen Bedeutungen wie oben beschrieben besitzen;
Entfernung der Schutzgruppe von der Aminogruppe des Indolskeletts unter Bildung einer Verbindung der Formel [XVII] 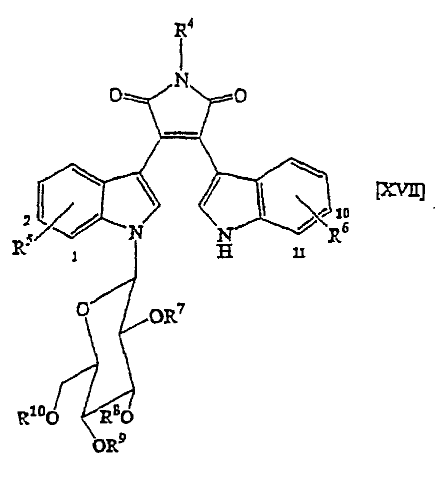 worin R⁴ bis R¹⁰ die gleichen Bedeutungen wie oben beschrieben besitzen;
Cyclisieren der Verbindung der Formel [XVII] mit Hilfe eines Oxidationsmittels unter Bildung einer Verbindung der Formel [XVIII] 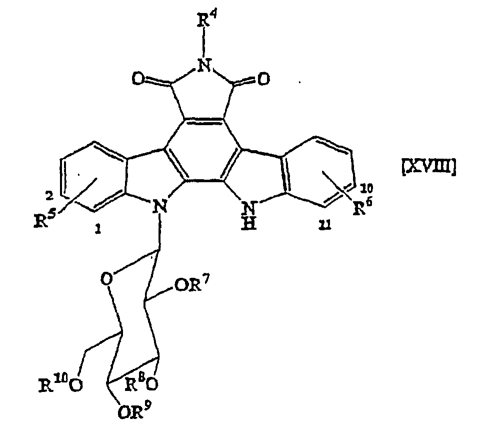 worin R⁴ bis R¹⁰ die gleichen Bedeutungen wie oben beschrieben besitzen;
Entfernung der Schutzgruppen für die OH-Gruppen unter Bildung einer Verbindung der Formel [XIX] 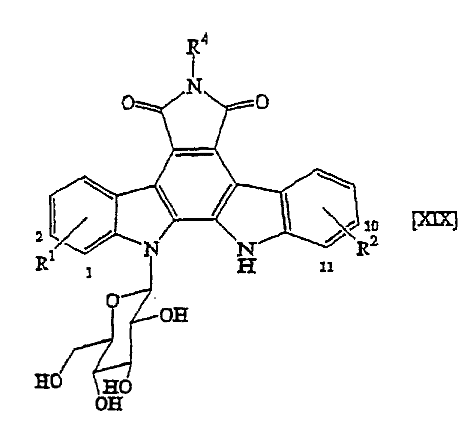 worin R¹ und R² je eine OH-Gruppe bedeuten und R¹ und R² in den 1- bzw. 11-Stellungen vorhanden sind oder R¹ bzw. R² in den 2- bzw. 10-Stellungen vorhanden sind; und R⁴ die gleichen Bedeutungen wie oben beschrieben besitzt;
Umsetzung der Verbindung der Formel [XIX] mit einer Base unter Bildung einer Verbindung der Formel [XX] 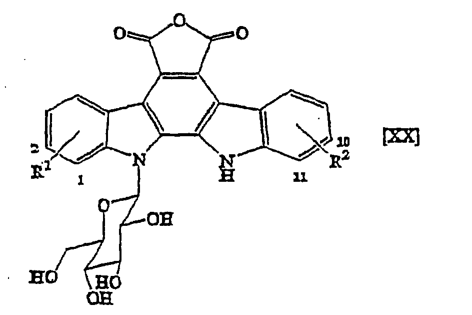 worin R¹ und R² die gleichen Bedeutungen wie oben beschrieben besitzen; und
Umsetzung der Verbindung der Formel [XX] mit H₂NNHCH(CH₂OH)₂.

5. Verbindung der Formel H₂NNHCH(CH₂OH)₂.

6. Antitumormittel, enthaltend eine Verbindung nach einem der Ansprüche 1 bis 3.

## Revendications

1. Composé de formule générale 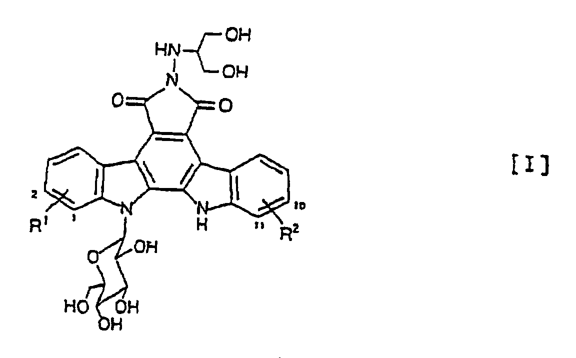 ou un sel pharmaceutiquement acceptable de celui-ci, où R¹ et R² représentent chacun un groupe OH, R¹ est situé à la position 1 ou 2, R² est situé à la position 10 ou 11, R² est situé à la position 11 lorsque R¹ est situé à la position 1, et R² est situé à la position 10 lorsque R¹ est situé à la position 2.

2. Composé de formule 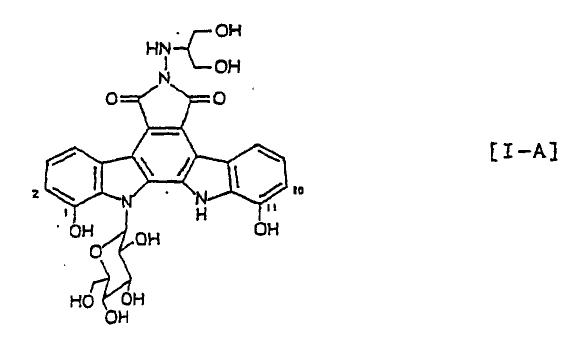 ou un sel pharmaceutiquement acceptable de celui-ci.

3. Composé de formule 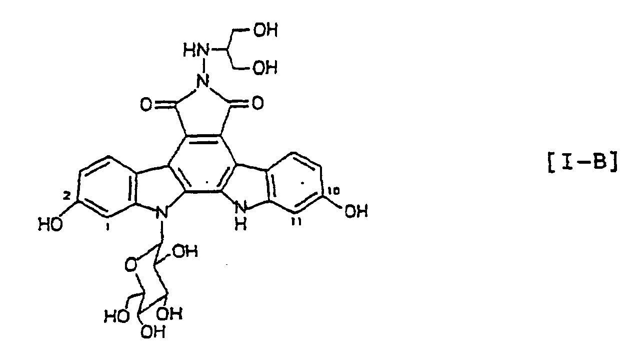 ou un sel pharmaceutiquement acceptable de celui-ci.

4. Procédé pour la préparation d'un composé de formule [I] 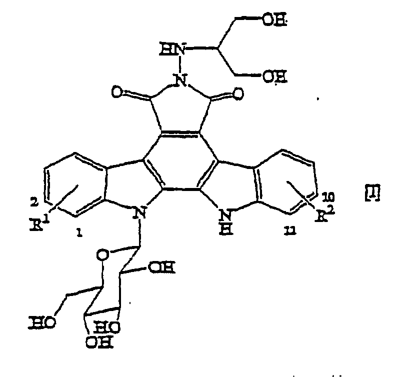 où R¹ et R² représentent chacun un groupe OH, R¹ est situé à la position 1 ou 2, R² est situé à la position 10 ou 11, R² est situé à la position 11 lorsque R¹ est situé à la position 1, et R² est situé à la position 10 lorsque R¹ est situé à la position 2, qui comprend :
la réaction d'un composé de formule [IX] 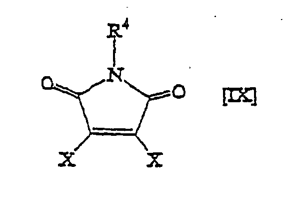 où R⁴ représente un atome d'hydrogène, un groupe alkyle ayant 1 à 6 atomes de carbone, un groupe benzyloxyméthyle ou un groupe aralkyle, et X représente un groupe partant,
avec un composé de formule [X] 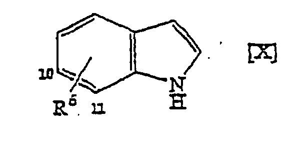 où R⁶ représente un groupe OH protégé, situé à la position 10 et 11,
en présence d'un composé organométallique pour former un composé de formule [XI] 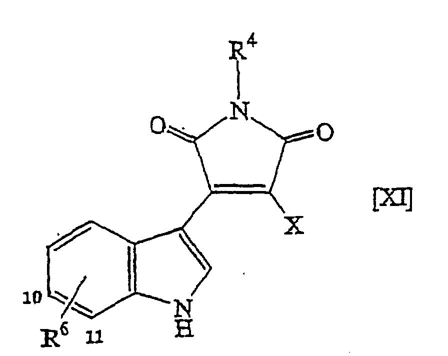 où R⁴, R⁶ et X sont tels que définis ci-dessus ;
la protection de l'atome d'hydrogène du groupe amino du squelette d'indole pour former un composé de formule [XII] 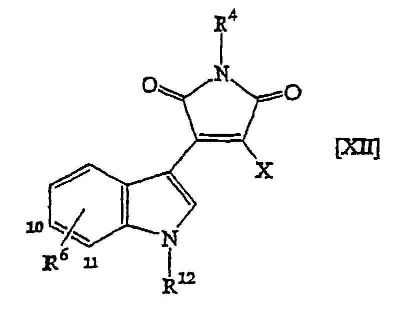 où R⁴, R⁶ et X sont tels que définis ci-dessus, et R¹² représente un groupe protecteur du groupe amino du squelette d'indole ;
la réaction du composé de formule [XII] avec un composé de formule [XIII] 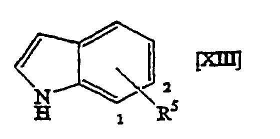 où R⁵ représente un groupe OH protégé à la position 1 ou 2,
en présence d'un composé organométallique, pour former un composé de formule [XIV] 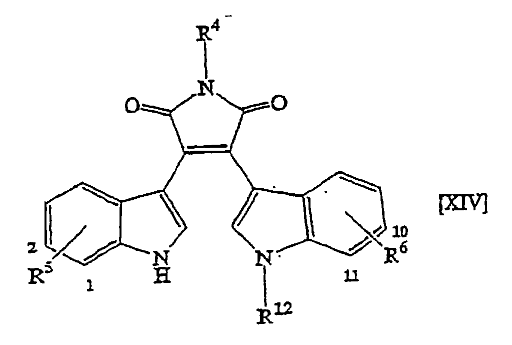 où R⁴, R⁵, R⁶ et R¹² sont tels que définis ci-dessus, et R⁵ et R⁶ sont situés aux positions 1 et 11, respectivement, ou sont situés aux positions 2 et 10, respectivement ;
la réaction du composé de formule [XIV] avec un composé de formule [XV] 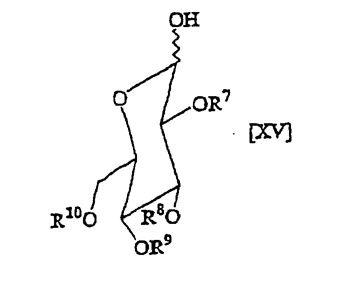 où R⁷, R⁸, R⁹ et R¹⁰ peuvent être identiques ou différents et représentent chacun un groupe protecteur d'un groupe hydroxyle,
par la réaction de Mitsunobu, pour former un composé de formule [XVI] 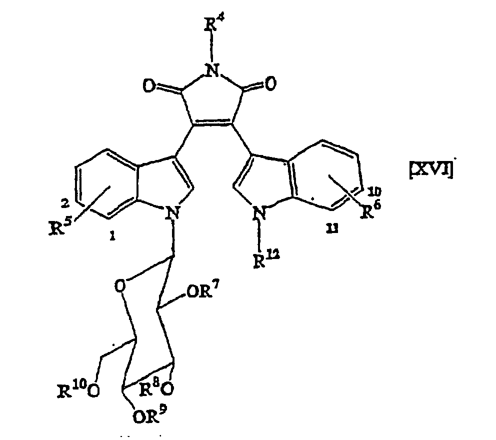 où R⁴ à R¹⁰ et R¹² sont tels que définis ci-dessus ;
l'élimination du groupe protecteur du groupe amino du squelette d'indole pour former un composé de formule [XVII] 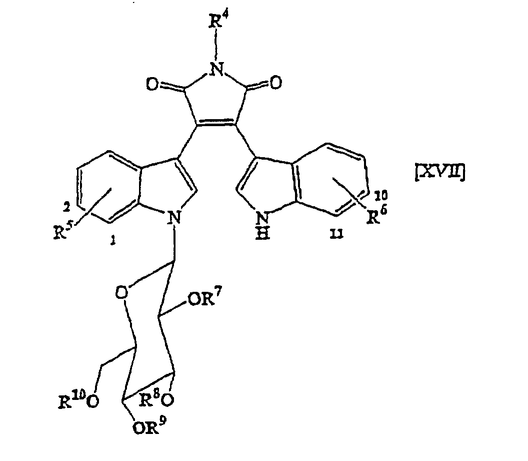 où R⁴ à R¹⁰ sont tels que définis ci-dessus ;
la cyclisation du composé de formule [XVII] à l'aide d'un agent oxydant pour former un composé de formule [XVIII] 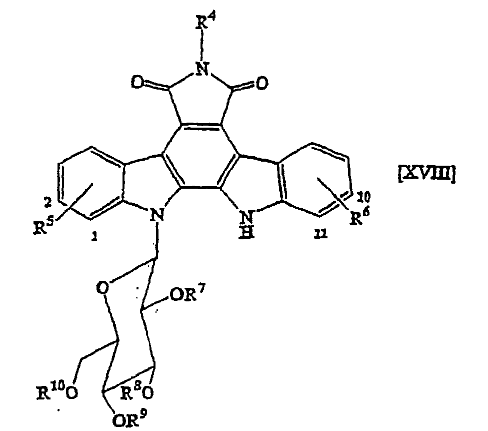 où R⁴ à R¹⁰ sont tels que définis ci-dessus ;
l'élimination des groupes protecteurs des groupes OH pour former un composé de formule [XIX] 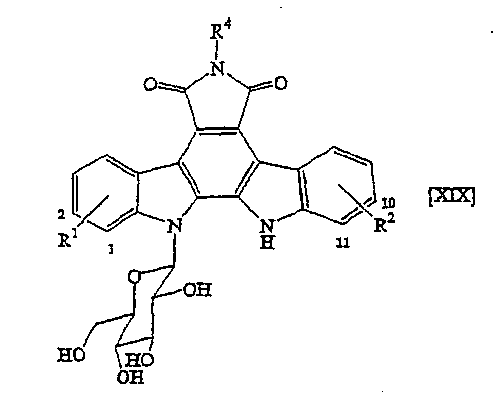 où R¹ et R² représentent chacun un groupe OH ; et R¹ et R² sont situés aux positions 1 et 11, respectivement, ou bien R¹ et R² sont situés aux positions 2 et 10, respectivement ; et R⁴ est tel que défini ci-dessus ;
la réaction du composé de formule [XIX] avec une base pour former un composé de formule [XX] 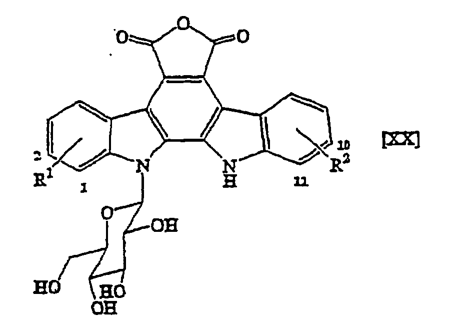 où R¹ et R² sont tels que définis ci-dessus ; et
la réaction du composé de formule [XX] avec H₂NNHCH(CH₂OH)₂.

5. Composé de f ormule H₂NNHCH(CH₂OH)₂.

6. Agent antitumoral contenant un composé tel que revendiqué dans l'une quelconque des revendications 1 à 3.
